(19) Europäisches Patentamt
European Patent Office
Office européen des brevets

(11) Publication number: **0 207 360 B1**

(12) **EUROPEAN PATENT SPECIFICATION**

(45) Date of publication of patent specification: **06.11.91**

(51) Int. Cl.$^{5}$: **G01N 33/52**, C12Q 1/54

(21) Application number: **86108238.6**

(22) Date of filing: **16.06.86**

# (54) Diagnostic test device.

(30) Priority: **28.06.85 US 749777**

(43) Date of publication of application:
**07.01.87 Bulletin 87/02**

(45) Publication of the grant of the patent:
**06.11.91 Bulletin 91/45**

(84) Designated Contracting States:
**DE FR GB IT SE**

(56) References cited:
**EP-A- 0 130 335**
**DE-B- 1 598 048**
**US-A- 3 006 735**
**US-A- 4 221 688**
**DE-A- 1 932 419**
**GB-A- 911 181**
**US-A- 3 979 274**
**US-A- 4 248 751**

(73) Proprietor: **MILES INC.**
**1127 Myrtle Street**
**Elkhart Indiana 46514(US)**

(72) Inventor: **Jones, James E.**
**2468 Camino Real S.**
**Virginia Beach, VA 23456(US)**

(74) Representative: **Dänner, Klaus, Dr. et al**
**c/o Bayer AG Konzernverwaltung RP Patentabteilung**
**W-5090 Leverkusen 1 Bayerwerk(DE)**

**EP 0 207 360 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

## Description

### Background of the Invention

#### A. Field of the Invention

The present invention relates to a new and improved test device for detecting glucose in fluids such as body fluids; and more particularly, to a new and improved testing device for detecting glucose in whole blood including a glucose permeable film of silicone water based elastomer over said device.

#### B. Description of the Prior Art

Products that measure fluctuations in a person's blood sugar, or glucose levels have become everyday necessities for many of the nation's seven million diabetics. Because this disorder can cause dangerous anomalies in blood chemistry and is believed to be a contributor to vision loss and kidney failure, most diabetics need to test themselves periodically and adjust their glucose count accordingly, usually with insulin injections. Patients who are insulin dependent - about 10% to 15% of all diabetics - are instructed by doctors to check their blood-sugar levels as often as four times daily.

For years the solution for diabetics was one of several urinanalysis kits that, despite repeated improvements, provided imprecise measurements of glucose in the blood. The first such kits used tablets. This early testing procedure is described in United States Patent Nos. 2,387,244 end 3,164,534. Later, reagent strips for urine testing were developed. Testing using urine, however, is limited in accuracy. The renal threshhold for glucose spillage is different for each individual. Moreover, sugar in urine is a sign that the sugar level is too high and has been too high for several hours. This is due to the delay in sugar reaching the urine.

More accurate readings are possible by taking readings from blood. The advent of home blood tests is considered by some to be the most significant advance in the care of diabetics since the discovery of insulin in 1921. Home blood glucose testing was made available with the development of reagent strips for whole blood testing. Reagent strips of this type are described in United States Patent Nos. 3,164,534 and 3,092,465. A breakthrough in self-care came in 1979, when the Ames division of Miles Laboratories brought out its Visidex home blood test. Visidex, consists of chemically coated plastic strips. When blood drawn by pricking a finger is placed on one of these disposable strips, the resulting color change can be compared with a color-coded glucose scale included in the kits or a reflectometer can be used.

One reagent strip test article used in the prior art for the detection of glucose in fluids is described in U.S. Patent No. 3,092,465. This prior art test article is particularly useful in detecting glucose in blood. It contains a test mixture impregnated in a bibulous material. The impregnated mixture contains glucose oxidase, peroxidase, o-tolidine dihydrochloride as an indicator, and a citric acid-sodium citrate buffer mixture. A semi-permeable coating, such as cellulose acetate, covers the impregnated portion of the bibulous material.

The principles underlying the basic reactions of enzyme tests for glucose are well known. Glucose oxidase catalyzes the aerobic oxidation of glucose to produce gluconic acid (gluconolactone) and hydrogen peroxide. In this reaction glucose reacts with atmospheric oxygen to form hydrogen peroxide. This reaction may be represented by the following schematic equation:

$$\text{Glucose}+O_2 \xrightarrow{\text{Glucose oxidase}} \text{Gluconic acid}+H_2O_2$$

A substance having peroxidative activity (such as horseradish peroxidase, iodide and molybdate salts, blood, etc.), in turn, is capable of inducing the oxidation of a class of indicators by the hydrogen peroxide formed in the conversion of glucose of glyconolcatone. This latter reaction may be represented by the following schematic equation:

$$H_2O_2 + \text{oxidizable dye} \xrightarrow[\text{}]{\text{Substance having peroxidative activity}} \text{Oxidized dye} + H_2O \text{ (color change)}$$

Using, as an example, materials known to the art, glucose oxidase, peroxidase and o-tolidine, the reaction proceeds as follows:

The glucose oxidase catalyzes the above reaction of oxygen with glucose present in the material being tested and there is formed, as indicated above, gluconic acid and hydrogen peroxide. The hydrogen peroxide in the presence of the peroxidase oxidizes the o-tolidine to its colored form, thus indicating the presence of glucose.

The previously described compositions of the prior art have the disadvantage that there has been a tendency for hemoglobin and other coloring bodies of whole blood to stain the test strip so that it assumes a dark red color which is interblended with the color developed on this stained strip by the glucose test composition forming a blended color which is difficult to read. As a result, color matching and determination after a smear test, especially for blood sugar, is beyond a normal or average person's ability to effect by mere visual perception.

To overcome this problem of staining, the strips or pads are coated with a semipermeable coating material in film form which screens out and prevents hemoglobin or protein materials from contacting the test composition. United States Patent No. 3,298,789 discloses a device of this type. Test strips prepared in this manner with a semipermeable layer forming material such as cellophane, cellulose acetate, cellulose butyrate, cellulose nitrate and the like allow water and glucose to pass through the coating material and react with the test composition on the strip while screening or preventing the larger molecules such as hemoglobin or other coloring matter as well as other protein constituents of the blood from passing through to the test composition. Since hemoglobin does not contact the test composition but remains separated therefrom by the coating film and remains on the outer surface, it may be wiped off with a tissue or washed from the coated strip with water. For example, if o-tolidine is present as the glucose indicator material, with the coating film the pure, unblended blue color of the oxidized o-tolidine is easily read from the strip and any variation in its intensity of color development is visually distinguishable. These variations in intensity form a clear visible index of the glucose concentration present in the original fluid tested and thus allow a simple chart based on this intensity phenomenon to be conveniently prepared for use in determining the concentration of glucose present in the tested sample.

A disadvantage of these prior art test devices is the coating or film is not sufficiently durable to withstand wiping to remove the larger molecules such as hemoglobin. Wiping often tears the coating or film ruining the test device and requiring another test to be performed. The prior art coating or film is also not sufficiently permeable to glucose which can affect the accuracy of test. It has also been discovered the material of the prior art film retains some of the larger molecules even after washing and additional wiping is required. DE-A-15 98 048 and DE-A-19 32 419, disclose test devices for the determination of glucose which are impregnated with solutions or emulsions of silicone.

## SUMMARY OF THE INVENTION

An object of the present invention is to provide a new and improved multilayer test device for detecting glucose in fluids such as body fluids.

Another object of the present invention is to provide a new and improved multilayer device for testing glucose in body fluids that can be wiped to improve visual perception of color changes in the device following contact with the fluid being tested.

A further object of the present invention is to provide a new and improved device for testing glucose in a fluid which device includes a film of varying thickness providing a color gradient upon contacting said device with fluid containing glucose.

Briefly, the present invention is directed to a new and improved multilayer device for detecting glucose in fluids such as body fluids comprising a first layer consisting of a liquid-retaining material impregnated with reagent capable of reacting with said glucose to produce a detectable change, and
a second layer coating the first layer with a selectively glucose-permeable, polymerized, silicone water-based elastomer. The test device can be rinsed and wiped to allow accurate visual reading of the indication

of the concentration of glucose in the sample being tested. The test device includes bibulous material impregnated with a mixture including an enzyme system with glucose oxidase activity, a substance having peroxidative activity, an indicator material which is oxidized in the presence of peroxide. The substance including peroxidative activity changes color. In accordance with the invention, bibulous strips or pads are impregnated with the composition and then dried. Once dried, the strips or pads are covered with a film preferably of a silicone water based elastomer that is glucose permeable. The film allows glucose to pass through to the bibulous material while screening larger molecules such as hemoglobin and proteins.

The film protects the bibulous material from contamination by any red coloration due to hemoglobin found in whole blood. The material of the film is sufficiently durable to allow rinsing and wiping to remove the larger molecules and hemoglobin from the top surface of the film. Rinsing and wiping improves visual perception of color change of the composition in the bibulous material. Improved perception is important since the variation of intensity of the color provides a visible index of glucose concentration present in the sample being tested. A mistaken perception of the color intensity could result in an incorrect diagnosis.

Prior art devices have the disadvantage that there is a tendency for the hemoglobin and other coloring bodies of whole blood to stain the bibulous material distorting the color intensity. Other devices have included a film but the film is of a material that is not easily cleaned to allow a proper reading and when wiped for cleaning, tends to tear destroying the device and requiring the test to be repeated.

If the test device includes side by side pads of bibulous material, the colors of adjacent pads tend to migrate between pads mixing the colors resulting in incorrect readings. The device of the present invention bonds the film over the pads and to the substrate between the pads preventing migration of colors between the pads.

## BRIEF DESCRIPTION OF THE DRAWINGS

The above and other objects and advantages and novel features of the present invention will become apparent from the following detailed description of the preferred embodiment of the invention illustrated in the accompanying drawings wherein:

FIG. 1 is a top plan view of a diagnostic test device constructed in accordance with the principles of the present invention; and

FIG. 2 is a view taken along line 2-2 in FIG. 1.

## DETAILED DESCRIPTION OF THE PREFERRED EMBODIMENTS

Referring to the drawing there is illustrated a diagnostic test device generally designated by the reference numeral 10. Test device 10 is intended for the detection of glucose in fluids, and especially in body fluids such as blood. Test device 10 includes bibulous material such as a strip or individual pads 12A and 12B which have been impregnated with a composition comprising an enzyme system having glucose oxidase activity, a substance having peroxidative activity (such as horseradish peroxidase, an iodide-molybdate salt, a urohemin and like substances having peroxidative activity), a color forming indicator (such as 2,7-diaminofluorene, o-tolidine, etc.), which is oxidizable in the presence of hydrogen peroxide and said substance having peroxidative activity.

Although, the bibulous material may be formed in a single strip and the body fluid applied to the strip for testing the concentration of glucose in the fluid, in the preferred embodiment illustrated, the bibulous material is formed into individual pads 12A and 12B. The pads 12A and 12B are secured on a substrate 14. Substrate 14 can be fabricated of an inexpensive, rigid material such as plastic allowing test device 10 to be easily handled. To allow a user to hold the diagnostic test device 10 during testing and reading, pads 12A and 12B are secured near a first end 16 of the device 10 and a second end 18 distant from the first end 16 defines a handle that can be gripped by the user.

Because the bibulous material is impregnated with, inter alia, a color forming indicator, a reading of the concentration of glucose in the sample fluid is made by visually comparing the color of the bibulous material with a chart. It occurs in prior art diagnostic test devices that a build up of red blood cells on the top surface of the pads 12A and 12B. The red coloring of these cells distorts the color change of the color forming indicator impregnated in the bibulous material making an accurate visual reading of the test device difficult or impossible. To overcome this problem, some prior art diagnostic test devices have included a semipermeable membrane coating superimposed over the bibulous material separating it from direct contact with the fluid specimen being tested. In making a test, these membrane coated strips are dipped in the fluid being tested. The red blood cells that would distort visual reading of color changes of the bibulous material are maintained on the top surface of the membrane. Any attached blood particles are wiped off or

washed off the membrane coating and the underlying strip shows variations in intensity forming a clear visible index of the glucose concentration present in the original fluid being tested. This allows a simple chart based on this intensity phenomenon to be conveniently prepared for use in determining the concentration of glucose present in the tested sample.

These prior art membrane coatings, however, allow the passage of water and when the diagnostic test device is rinsed, some rinse water passes through the membrane washing some of the color from the pads. In addition, to remove the red blood cells completely from the surface of the membrane, wiping is necessary. The material of prior membranes is cellulose acetate or similar material and is not suitable for wiping because it tears easily. If the coating is torn, the test must be repeated. A further problem occurs with prior art test devices due to the coating not being three dimensional. The coating is typically provided in sheets that are applied over the bibulous material and cut. Cutting exposes the sides of the device and prevents a three dimensional seal entirely around the bibulous material.

In the diagnostic test device 10 of the present invention these problems are overcome by a glucose permeable membrane, overcoat or film 20. The overcoat or membrane 20 is a silicone water based elastomer that is a liquid in the precured state. The silicone water based elastomer overcoat 20 is glucose permeable and this characteristic allows it to be used to allow passage of glucose while screening larger molecules such as red blood cells.

In accordance with an important feature of the present invention it has been found that a dispersion of a polymerizable silicone water-based elastomer applied in an incompletely cured form as a dispersed phase in an aqueous carrier, the silicone water-based elastomer being essentially insoluble in the said carrier liquid and a portion of the said carrier liquid removable from the dispersion during curing, will dry and cure as a continuous layer, film or membrane having unexpectedly high glucose-permeability to function as the overcoat 20. The the silicone water-based elastomer can be dispersed in the continuous phase as a monomer, oligomer, pre-polymer, or incompletely cured polymer. The the silicone water-based elastomer is cured in place as a continuous polymeric coating or layer. The removable aqueous carrier removed during curing, such as by volatilization, should be included in an amount of at least 5% by weight of the dispersion, and preferably 10-90% by weight.

It has been found that the polymerizable the silicone water-based elastomer including monomers, oligomers, pre-polymers, and incompletely cured polymers or mixtures thereof capable of polymerization or further polymerization in dispersed form will form cured layers or membranes when cured or polymerized in a dispersed layer upon removal of the continuous phase during curing to provide a layer or membrane having unexpectedly good oxygen and glucose-permeability without allowing the passage of interferants therethrough. The polymerizable silicone elastomers after dispersion in a continuous phase, such as by including an emulsifier, can be cured in any known manner during removal of the continuous phase, such as by evaporation of water from a water-continuous phase silicone emulsion or dispersion, as disclosed in Johnson et al Patent No. 4,221,688, or as disclosed in Elias Patent No. 4,427,811. Further, the dispersion of the silicone-elastomer compound compound can include a suitable curing catalyst or can be heat cured so long as the dispersion of the polymerizable silicone elastomer is applied as a layer in the form of an incompletely cured dispersion and at least a portion of the aqueous carrier or continuous phase is removed from the dispersion during final curing. Without being limited to any particular mechanism, it is theorized that some alignment of the aggregating or polymerizing silicone elastomer molecules, during polymerization, occurs during final removal of the carrier to form micelles such that the aggregating silicone elastomer molecules are bound upon curing in a manner capable of permitting the permeation of glucose and oxygen between molecules while excluding electrode-sensitive interferants.

The silicone elastomer compounds, useful in accordance with the invention are those which are essentially insoluble in water, are polymerizable in the dispersed form, and result in a continuous film or layer upon curing.

In accordance with one embodiment of the present invention, the polymerizable silicone compound is an organosiloxane, and particularly a diorganosiloxane comprising essentially a linear species of repeating diorganosiloxane units which may include small numbers of monoorganosiloxane units up to a maximum of about one monoorganosiloxane unit for each 100 diorganosiloxane units wherein the polymer chain is terminated at each end with silicone-bonded hydroxyls, as disclosed in Johnson et al. U.S. Patent No. 4,221,688.

In accordance with another important embodiment of the present invention, the polymerizable water-based silicone elastomer forming a glucose-permeable overcoat or membrane is applied onto a substrate 14 as an aqueous silicone emulsion comprising a continuous water phase and an anionically stabilized dispersed silicone phase wherein the silicone phase is a graft copolymer of a water soluble silicate and a hydroxyl endblocked polydiorganosiloxane. As disclosed in the Saam Patent No. 4,244,849, such silicone

emulsions having a pH within the range of from 8.5 to 12, are stable upon extended storage and result in a cured elastomeric continuous layer upon removal of water under ambient conditions. These silicone compounds are obtained from the interaction of hydroxyl end-blocked polydiorganosiloxanes and alkali metal silicates to form graft copolymers anionically stablized in aqueous emulsions at a pH of, for example, 8.5 to 12. If stability is not important, however, the pH is not critical. For example, the emulsion can be applied in layer form to manufacture the membrane as soon as the components are homogeneously dispersed.

The expression "hydroxyl endblocked polydiorganosiloxane" is understood to describe an essentially linear polymer of repeating diorganosiloxane units containing no more than small impurities of monoorganosiloxane units. The hydroxyl endblocked diorganosiloxane will therefore have essentially two silicon-bonded hydroxyl radicals per molecule. To impart elastomeric properties to the product obtained after removal of the water from the emulsion, the polysiloxane should have a weight average molecular weight ($M_w$) of at least 5,000. Polysiloxanes with weight average molecular weights below 5000, for example down to about 90, also are useful so long as the polymers form a continuous film or layer upon curing. Tensile strengths and elongations at break improve with increasing molecular weight with relatively high tensile strengths and elongations obtained above 50,000 $M_w$. The maximum $M_w$ is one which can be emulsified or otherwise dispersed in a liquid carrier or continuous phase, such as water. Weight average molecular weights up to about 1,000,000 for the incompletely cured dispersed polysiloxane are expected to be practical for this invention. Upon curing, there is no upper limit to the molecular weight of the overcoat or membrane. The preferred $M_w$ for the polymerizable dispersed siloxane is in the range of 1,000 to 700,000.

Organic radicals on useful hydroxyl endblocked polydiorganosiloxanes can be, for example, monovalent hydrocarbon radicals containing less than seven carbon atoms per radical and 2-(perfluoroalkyl)ethyl radicals containing less than seven carbon atoms per radical. Examples of monovalent hydrocarbon radicals include methyl, ethyl, propyl, butyl, isopropyl, pentyl, hexyl, vinyl, cyclohexyl and phenyl and examples of 2-(perfluoroalkyl)ethyl radicals include 3,3,3-trifluoropropyl and 2-(perfluorobutylmethyl). The hydroxyl endblocked polydiorganosiloxanes preferably contain organic radicals in which at least 50 percent are methyl. The preferred polydiorganosiloxanes are the hydroxyl endblocked polydimethylsiloxanes.

In accordance with one important embodiment of the present invention, the hydroxyl endblocked polydiorganosiloxane is employed as an anionically stabilized aqueous emulsion. For the purposes of this embodiment "anionically stabilized" means the polydiorganosiloxane is stabilized in emulsion with an anionic surfactant. The most preferred anionically stabilized aqueous emulsion of hydroxyl endblocked polydiorganosiloxane are those prepared by the method of anionic emulsion polymerization described by Findlay et al. in U.S. Patent No. 3,294,725 showing the methods of polymerization and showing anionically stabilized emulsions of hydroxyl endblocked polydiorganosiloxanes. Another method of preparing hydroxyl endblocked polydiorganosiloxanes is described by Hyde et al in U.S. Patent No. 2,891,920, which discloses the hydroxyl endblocked polydiorganosiloxanes and their method of preparation. These methods and others are known in the art.

An alkali metal silicate or colloidal silica can be included in the emulsified silicone composition for the preparation of extended storage stable emulsions used in the invention. The alkali metal silicates preferred for use in the emulsions forming the glucose-permeable membranes of the present invention are water soluble silicates. The alkali metal silicate is preferably employed as an aqueous solution. Aqueous silicate solutions of any of the alkali metals can be employed such as lithium silicate, sodium silicate, potassium silicate, rubidium silicate and cesium silicate.

The colloidal silicas are well known in the art and many are commercially available and can be included in the dispersion for increased strength and storage stability. Although any of the colloidal silicas can be used including fumed colloidal silicas and precipitated colloidal silicas, the preferred colloidal silicas are those which are available in an aqueous medium. Colloidal silicas in an aqueous medium are usually available in a stabilized form, such as those stabilized with sodium ion, ammonia or an aluminum ion. Aqueous colloidal silicas which have been stabilized with sodium ion are particularly useful for forming an emulsion because the pH requirement can be met by using such a sodium ion stabilized colloidal silica without having to add additional ingredients to bring the pH within the range of, for example, 8.5 to 12. The expression "colloidal silica" as used herein is those silicas which have particle diameters of from 0.0001 to 0.1 micrometers. Preferably, the particle diameters of the colloidal silicas are from 0.001 to 0.05 micrometers.

The colloidal silica can be added to the anionically stabilized hydroxylated polydiorganosiloxane in the form of a dry powder or as an aqueous dispersion. The best method is to add the colloidal silica in the form of a sodium ion stabilized aqueous dispersion of colloidal silica. There are many such sodium ion stabilized aqueous dispersions of colloidal silica which are commercially available. These commercial colloidal silicas

are usually available in aqueous dispersions having from 15 to 30 weight percent colloidal silica and having a pH in the range of 8.5 to 10.5.

Aqueous solutions of sodium or potassium silicate are well known and are commercially available. The solutions generally do not contain any significant amount of discrete particles of amorphous silica and are commonly referred to as water glass. The ratio by weight of $SiO_2$ to alkali metal oxide in the aqueous solutions of alkali metal silicates is not critical and may be varied within the usual range of about 1.5 to 3.5 for the sodium silicates and 2.1 to 2.5 for the potassium silicates. The aqueous alkali metal silicate solutions are particularly useful in preparing the emulsions of the present invention because the addition of the silicate solution often brings the pH of the emulsion within the range of about 8.5 to about 12 so that additional ingredients are not necessary to adjust the pH of the emulsion. Of course, other aqueous alkali metal silicate solutions such as those prepared by hydrolyzing silicon esters in aqueous alkali metal hydroxide solutions can also be employed in the present invention.

In accordance with one embodiment of the present invention, the polymerizable silicone containing compound is dispersed by combining an aqueous solution of an alkali metal silicate and the polymerizable silicone compound in an emulsion so that a graft copolymer is formed as dispersed particles. The preferred procedure for preparing silicone emulsions is to add the alkali metal silicate to an anionically stabilized aqueous emulsion of one or more hydroxyl endblocked polydiorganosiloxanes, adjust the pH of the emulsion within the range of about 8.5 to 12 and then age the emulsion for a time period such that an elastomeric product is formed upon removal of the water under ambient conditions. In this embodiment, the pH of the emulsion containing dissolved silicate and dispersed hydroxyl endblocked polydiorganosiloxane is important to the formation of the emulsion. A pH of 8.5 to 12 maintains the alkali metal silicate dissolved so that sufficient graft copolymerization between the dissolved silicate and dispersed siloxane occurs during removal of the carrier (e.g. water) to produce an emulsion capable of providing polymerization or further polymerization of the silicon-containing compound when deposited as a layer to form a membrane. If the pH is lower than the stated range, silicic acid is formed from the alkali metal silicate. Silicic acid is unstable and rapidly polymerizes by condensation which can gel the emulsion. Since silicic acid formation is almost completely suppressed at a pH of 10 to 12 and the reaction between dissolved alkali metal silicate and dispersed siloxanes occurs more rapidly within the pH range of 10-12, this pH range is preferred for emulsions containing an alkali metal silicate.

Silicone emulsions prepared by this silicate copolymerization embodiment are aged at a pH range of 8.5 to 12 for a time period sufficient to allow interaction between the dissolved silicate and the dispersed siloxane so that an elastomeric product is formed upon removal of the water under ambient conditions, as disclosed in Saam U.S. Pat. No. 4,244,849. The aging period is effectively reduced when an organic tin salt is employed in an amount of about 0.1 to 2 parts by weight for each 100 parts by weight of polydiorganosiloxane. The organic tin salts expected to be useful in the emulsions include mono-, di- and tricrganotin salts. The anion of the tin salt employed is not critical and can be either organic or inorganic although organic anions such as carboxylates are generally preferred. Organic tin salts that can be employed include octyltin triacetate, dioctyltin dioctoate, didecyltin diacetate, dibutyltin diacetate, dibutyltin dibromide, dioctyltin dilaurate and trioctyltin acetate. The preferred diorganotin dicarboxylate is dioctyltin dilaurate.

The concentration of the polymerizable silicone compound, e.g. the hydroxyl endblocked polydiorganosiloxane in the stabilized emulsion is not critical, particularly since the water or other continuous phase carrier is removed during curing of the Si phase during film, layer or membrane formation.

The relative amounts of alkali metal silicates and hydroxyl endblocked polydiorganosiloxane employed can vary over a considerable range. Preferred elastomer properties are obtained when 0.3 to 30 parts by weight silicate is employed for each 100 parts by weight siloxane.

Other useful polymerizable silicone compounds for forming the dispersions useful in forming a continuous elastomeric silicone polymer membrane having glucose-permeability in accordance with the present invention include the vinyl endblocked polydiorganosiloxanes dispersed together with an organosilicone compound having silicon-bonded hydrogen atoms, as disclosed in the Willing Patent No. 4,248,751. As disclosed in the Willing patent, these silicone compounds are generally dispersed by emulsifying the vinyl endblocked polydiorganosiloxane together with an organosilicone compound having silicon-bonded hydrogen atoms using water and a surfactant to form an emulsion and thereafter adding a platinum catalyst and heating the emulsion to form a cross-linked silicone.

The vinyl endblocked polydiorganosiloxane can be any of the polydiorganosiloxanes endblocked with diorganovinylsiloxy units and can be represented by the formula

$$(CH_2=CH)R_2SiO(R_2SiO)_xSiR_2(CH=CH_2)$$

where each R is a monovalent hydrocarbon radical or a monovalent halogenated hydrocarbon radical and x is a representation of the number of repeating diorganosiloxane units in the polymer. The monovalent radicals can be any of those known in the art, but are preferably those with six carbon atoms or less. The preferred polydiorganosiloxanes are those wherein the monovalent organic radicals are methyl, ethyl, phenyl, 3,3,3-trifluoropropyl and mixtures thereof wherein at least 50 percent of the radicals are methyl radicals. The polydiorganosiloxane can be a single type polymer with the same kind of repeating diorganosiloxane units or with a combination of two or more kinds of repeating diorganosiloxane units, such as a combination of dimethylsiloxane units and methylphenylsiloxane units. A mixture of two or more polydiorganosiloxanes also is useful. The value of x is not critical since upon final curing in the dispersed layer, the value of x increases rapidly. The upper limit of polydiorganosiloxane which is suitable for this invention is limited only to the extent that it cannot be dispersed to form a homogenous dispersion to achieve a homogenous layer capable of forming a continuous membrane upon complete curing.

In accordance with this vinyl-endblocked embodiment, the organosilicon compound or mixture of compounds dispersed with the polydiorganosiloxane is one which contains silicon-bonded hydrogen atoms. The organosilicon compound can be any compound or combination of compounds containing silicon-bonded hydrogen atoms useful as crosslinkers and providing an average of silicon-bonded hydrogen atoms per molecule of organosiloxane compound of at least 2.1. Such organosilicon compounds are known in the art as illustrated in U.S. Pat. No. 3,697,473. The preferred organosilicon compounds are those which are siloxanes made up of units selected from $HSiO_{1.5}$, R'HSiO, $R'_2HSiO_{0.5}$, $R'SiO_{1.5}$, $R'_2SiO$, $R'_3SiO_{0.5}$ and $SiO_2$ such that there is at least 2.1 silicon-bonded hydrogen atoms per molecule. Each R' is preferably selected from an alkyl radical of 1 to 12 carbon atoms inclusive, phenyl and 3,3,3-trifluoropropyl.

The amount of vinyl endblocked diorganosiloxane and organosilicon compound can vary broadly in weight amounts because the unit of weight for each vinyl radical or silicon-bonded hydrogen atom will vary considerably. Such "units of weight" are determined by dividing the molecular weight by the number of vinyl radicals per molecule or number of SiH per molecule. Because the cross-linked molecules in the membrane are formed by the reaction between the vinyl radical of the polydiorganosiloxane and the silicon-bonded hydrogen atom (SiH) of the organosilicon compound, the amounts of each will depend upon the ratio of SiH to vinyl. The stoichiometry would suggest that about one SiH per vinyl is all that is needed, however, the reactivity of the SiH can vary significantly, as well as its availability for reaction. For this reason, the ratio of SiH to vinyl can vary beyond the stoichiometric amounts and still provide products capable of polymerizing in layer form to provide continuous glucose-permeable overcoats or membranes. The vinyl endblocked polydiorganosiloxane and organosilicon compound preferably are combined such that the ratio of SiH to vinyl can vary from 0.75/1 to 4/1, with the most preferred range of 0.75/1 to 1.5/1.

The platinum catalyst can be any of the platinum catalysts known to catalyze the addition of silicon-bonded hydrogen atoms to silicon-bonded vinyl radicals. Platinum catalysts can be any of the known forms, ranging from platinum as such or as deposited on carriers such as silica gel or powdered charcoal, to platinic chlorides, salts of platinum and chloroplatinic acid. The dispersibilty of the platinum catalysts in the siloxane can be increased by complexing it with vinyl-containing siloxanes such as described in U.S. Pat. No. 3,419,593.

The amount of platinum catalyst used should be such that there is at least 0.1 part by weight platinum per one million parts by weight of the combined weight of polydiorganosiloxane and organosilicon compound. Preferably, the amount of catalyst used is from 1 to 20 parts by weight platinum per million parts by weight of polydiorganosiloxane and organosilicon compound. Larger amounts of platinum can be used if economic considerations are not important.

For those cases where a platinum catalyst is included in the dispersion and a platinum catalyst inhibitor is desired to prevent complete curing prior to layering the dispersion for formation of the overcoat or membrane, there are many types of known inhibitors. These inhibitors retard or inhibit the activity of the platinum catalyst, but allow the platinum catalyst to become active at elevated temperatures, such as above 70° C. If the carrier in the dispersion is water, the selection of an inhibitor should be one which does not have its effectiveness destroyed by water or surfactants or it does not destroy the emulsion. Effective inhibitors include the acetylenic alcohols and other acetylenic compounds described in U.S. Pat. No. 3,445,420. Other platinum catalyst inhibitors are known as defined in U.S. Pat. No.3,188,299, U.S. Pat. No. 3,188,300, U.S. Pat. No. 3,192,181, U.S. Pat. No. 3,344,111, U.S. Pat. No. 3,383,356, U.S. Pat. No. 3,453,233, U.S. Pat. No. 3,453,234 and U.S. Pat. No. 3,532,649. The dispersed composition can be heated for a period of time to partially cross-link the Si-containing compounds to form a stable emulsion of cross-linked particles dispersed in a carrier. After application in layer form on a substrate, the layer further cures to form a continuous, glucose permeable overcoat or membrane.

Evaporation of the carrier may be assisted by a flow of dry air or other gas, either at ambient temperature or at an elevated temperature, by infrared heating or a combination of the various means. Care should be taken when accelerated means are used to evaporate the carrier, e.g. water, that the rapidly leaving water vapor does not produce undesirable discontinuities in the film.

Other reinforcing materials useful for increasing the structural integrity of the cured glucose-permeable membranes of the present invention include the copolymers disclosed in the Huebner et al Patent No. 4,288,356. The copolymers are emulsion polymerized and comprise free radical polymerized monomers selected from at least one unsaturated organic monomer and at least one unsaturated organosilicone monomer. The copolymers are made from 1 to 7 weight percent unsaturated organosilicon monomer and from 93 to 99 weight percent organic monomer. It is believed that any of the unsaturated organic monomers commonly used to form polymers through free radical polymerization can be used either by themselves or in combination; for example, styrene, methylmethacrylate, and vinyl chloride. The unsaturated organosilicon monomer can be an unsaturated silane, siloxane, or silazane that will copolymerize with the unsaturated organic monomer or mixture of unsaturated organic monomers used and will form SiOH under the conditions of an emulsion polymerization method used to produce the copolymer.

The unsaturated organosilicon monomer can be a silane of the formula $R'R''_xSi(R''')_{3-x}$ where R' is an olefinic unsaturated radical such as vinyl, allyl, acryloxypropyl, or methacryloxypropyl, R" is an alkyl radical containing 1 to 4 inclusive carbon atoms or a phenyl radical, and R''' is a hydrolyzable group such as -OR", - OCOR", or halogen, and x is 0, 1 or 2. The unsaturated organosilicon monomer can be a cyclic siloxane of the formula (R'R"SiO)a where R' and R" are as defined and a is from 3 to 6 inclusive. The unsaturated organosilicon monomer can be a disilazane of the formula $R'R''_2Si\text{-}NH\text{-}SiR''_2R'$ where R' and R" are as defined. The unsaturated organosilicon monomer can be a cyclic silazane of the formula $(R'R''SiNH)_3$ where R' and R" are as defined. A preferred unsaturated organosilicon monomer is vinyltriethoxysilane.

Examples of unsaturated organosilicon monomers include silanes such as $ViMeSiCl_2$, $ViMe_2SiOMe$, $ViMeSi(OEt)_2$, and $ViSi(OEt)_3$,siloxanes such as $(ViMe_2Si)_2O$, $(ViMeSiO)_3$, and (ViMeSiO)a where a is 3 to 6 inclusive, and silazanes such as $(ViMe_2Si)_2NH$ and $(ViMeSiNH)_3$ where Me is methyl radical, E+ is the ethyl radical and Vi is vinyl radical.

The unsaturated organic monomer and the unsaturated organosilicon monomer can be emulsion polymerized by the common methods of performing such copolymerizations. One such process is described by Blackderf in U.S. Pat. No. 3,706,697 disclosing a process for copolymerizing an acrylic ester and an acryloxyalkylalkoxysilane by emulsion polymerization of the organic monomer through a free radical generator.

For example, a mixture is prepared of water and an anionic surfactant, and then a mixture of styrene and vinyltriethoxysilane is slowly added under a nitrogen blanket. Ammonium persulfate then is added as the polymerization catalyst. Heating the mixture initiates the polymerization, but it is also necessary to control the reaction temperature so that the emulsion does not overheat due to the exothermic reaction. After polymerization, the emulsion is adjusted to a pH of greater than 7.

The copolymer is added in amount of 5 to 100 parts (pph) by weight of the emulsion polymerized copolymer for each 100 parts by weight of polymerizable silicone compound, e.g. polydiorganosiloxane. The addition of the copolymer serves to act as a reinforcement or filler for the polydiorganosiloxane. Amounts of from 5 to 25 parts of copolymer added per 100 parts of polymerizable Si-containing compound yield a reinforced overcoat or membrane having the desired glucose-permeability and strength without the addition of other fillers such as $SiO_2$. When the amount of copolymer added is from 25 to 60 parts by weight, the final product obtained by drying the emulsion is a higher strength membrane. The more copolymer added, the harder and less elastic the final overcoat or membrane becomes.

In accordance with one embodiment of the invention, an alkyl tin salt is added to the dispersion to catalyze the curing of the final emulsion during the devolatilization or other removal of the carrier to yield the cured overcoat or membrane. Preferred salts are dialkyltin dicarboxylates such as dibutyltindiacetate, dibutyltindilaurate, and dioctyltindilaurate. Most preferred is dibutyltindilaurate. The emulsion of catalyst is used in an amount sufficient to yield from 0.1 to 2 parts by weight of the alkyl tin salt for each 100 parts by weight of the polymerizable silicone compound, e.g. polydiorganosiloxane. Larger amounts could be used, but the larger amount would serve no useful purpose.

A silane cross-linking agent, of the general formula $A_m\text{-}Si(OR)_{4-m}$ can be added to the dispersion to enhance the physical properties of the cured overcoat or membrane. The radical A, in the silane cross-linking agent is a member selected from the group consisting of a hydrogen atom, monovalent hydrocarbon radicals containing 1 to 6 inclusive carbon atoms, and monovalent halohydrocarbon radicals containing 1 to 6 inclusive carbon atoms. Preferred radicals are methyl, ethyl, phenyl, and 3,3,3-trifluoropropyl with methyl being most preferred. The radical R is a hydrogen atom, and alkyl group containing 1 to 4 inclusive carbon

atoms,

$$-\overset{O}{\overset{\|}{C}}CH_3, \qquad -\overset{O}{\overset{\|}{C}}C_2H_5,$$

$-CH_2CH_2OH$, $-CH_2CH_2OCH_3$, or a $-CH_2C\text{-}H_2OC_2H_5$ group. The R radicals on a silane molecule can be the same or different. The number of A radicals can be 0 or 1, meaning that a silane molecule can be either tri or tetra-functional in order to function as a cross-linker in the curing of the final overcoat or membrane of this invention. The OR group on the silane is a hydrolyzable group that forms $\equiv$SiOH during curing of the membranes of this invention. The preferred silane cross-linking agent is methyltrimethoxysilane. The silane crosslinking agent can be included in a sufficient amount to obtain the desired degree of crosslinking. The amount to be used depends upon the hydroxyl content of the polymerizable silicone compound and the molecular weight of the crosslinking agent chosen. The more crosslinking agent used, the harder and less elastic the membrane becomes. Useful amounts of the preferred methyltrimethoxysilane cross-linker vary from 1 to 7 parts by weight of silane per 100 parts by weight of polydiorganosiloxane.

Other useful silicone containing compounds capable of polymerizing to form a membrane, film or layer that is glucose-permeable include the copolymers of diorganosiloxanes and any hydrolyzable silane, as disclosed in the Sorkin Patent No. 3,624,017.

The diorganosiloxanes can be included in the dispersion as a monomer or a polymer. The monomer can be partially polymerized in the dispersion or emulsion and then silane added and copolymerized with the diorganosiloxane polymer. The surfactant used to form an emulsion with the copolymers can be either anionic, cationic or nonionic and any catalyst useful to initiate the copolymerization can be used, such as a strong acid or a strong base. The starting diorganosiloxane can be either a cyclic or a linear material and the molecular weight of the starting diorganosiloxane is not critical.

The dispersion of the polymerizable silicone compound or compounds can contain the components in a broad range of concentrations. The preferred concentration range will depend on the thickness of the overcoat or membrane desired. For example, to provide a thick elastomeric overcoat that does not form cracks as the carrier or continuous phase evaporates, it is best to use a dispersion having a combined amount of silicate and polydiorganosiloxane in the range of 67 to 160 parts by weight for each 100 parts by weight of carrier, e.g. water. Preferred overcoat thicknesses are 0.013 to 0.64 mm (0.5 to 25 mils), for example 0.11 mm (4.5 mils).

If an emulsifying agent is incorporated into the composition to form the dispersion the amount of emulsifying agent can be less than 2 weight percent of the emulsion, and the emulsifying agent can result from neutralized sulfonic acid used in the emulsion polymerization method for the preparation of a hydroxyl endblocked polydiorganosiloxane.

Anionic surfactants are preferably the salt of the surface active sulfonic acids used in the emulsion polymerization to form the hydroxyl endblocked polydiorganosiloxane as shown in U.S. Pat. No. 3,294,725 disclosing the surface active sulfonic acids and salts thereof. The alkali metal salts of the sulfonic acids are preferred, particularly the sodium salts. The sulfonic acid can be illustrated by aliphatically substituted benzenesulfonic acids, aliphatically substituted naphthalene sulfonic acids, aliphatic sulfonic acids, silylalkylsulfonic acids and aliphatically substituted diphenylethersulfonic acids. Other anionic emulsifying agents can be used, for example, alkali metal sulforicinoleates, sulfonated glyceryl esters of fatty acids, salts of sulfonated monovalent alcohol esters, amides of amino sulfonic acid such as the sodium salt of oleyl methyltauride, sulfonated aromatic hydrocarbon alkali salts such as sodium alpha-naphthalene monosulfonate, condensation products of naphthalene sulfonic acids with formaldehyde, and sulfates such as ammonium lauryl sulfate, triethanol amine lauryl sulfate and sodium lauryl ether sulfate.

Nonionic emulsifying agents also can be included in the emulsion in addition to the anionic emulsifying agents. Such nonionic emulsifying agents are, for example, saponins, condensation products of fatty acids with ethylene oxide such as dodecyl ether of tetraethylene oxide, condensation products of ethylene oxide and sorbitan trioleate, condensation products of phenolic compounds having side chains with ethylene oxide such as condensation products of ethylene oxide with isododecylphenol, and imine derivatives such as polymerized ethylene imine.

The polymerizable silicone dispersion used to form the glucose-permeable membranes of the present invention can contain additional ingredients to modify the properties of the dispersions or the cured polymeric membrane products obtained from the dispersion. For example, a thickener can be added to modify viscosity of the dispersion or to provide thixotropy for the dispersion. An antifoam agent can be

added to the dispersion to reduce foaming during preparation, coating or curing in layer form.

Fillers can be added to the dispersion to reinforce, extend or pigment the overcoat or membrane. Useful fillers include colloidal silica, carbon black, clay, alumina, calcium carbonate, quartz, zinc oxide, mica, titanium dioxide and others well known in the art. These fillers should be finely divided and it may be advantageous to use aqueous dispersions of such fillers if they are commercially available, such as aqueous dispersions of carbon black. The polymerizable Si-compound containing dispersions do not require a filler and such can be added in dry or aqueous forms to provide selected properties to the membrane.

The filler preferably has an average particle diameter of less than 10 micrometers. Useful fillers have had average particle diameters ranging down to as low as 0.05 micrometers. When these silicone emulsions are spread out for final curing to form the glucose-permeable overcoats of the present invention, the water evaporates, or is otherwise removed, to leave a cured glucose and oxygen-permeable overcoats. Evaporation of the carrier is usually complete within a few hours to about one day depending on the dispersion film thickness and method of application. Another of the important advantages of the present invention is the excellent adhesion shown by these overcoats for both polar and nonpolar substrates.

It should be understood that this invention is not limited to removal of continuous liquid phase in the silicone dispersion by evaporation, since other methods such as coagulation may be useful. Heating the polymerizable silicon-containing dispersions to more rapidly remove the acquous carrier to produce more rapidly cured overcoats also may be advantageous.

The preferred materials for overcoat or membrane 20 are an anionically stabilized, water-based hydroxyl endblocked polydimethylsiloxan elastomer containing about 5 percent by weight colloidal silica sold by Dow Corning as elastomer and manufactured in accordance with Dow Corning U.S. Pat. No. 4,221,668. To show the new and unexpected results using overcoats or membranes 20 formed from a dispersion of polymerizable silicon-containing compounds applied in layer form in an incompletely cured state dispersed in a removable liquid carrier, four membranes were made-three from silicone-water liquid dispersions and one from a silicone paste material having essentially no removable liquid phase. The membranes were prepared by casting the elastomers onto a polyester film with a 0.25 mm (10 mil) doctor blade and curing at ambient conditions. The three compositions having a removable carrier (water) were applied as a neat polysiloxane emulsions. Curing was accomplished in 30-60 minutes, but can be accelerated with heat. This process gave a final dry film (membrane) thickness of approximately 0.11 mm (4.5 mils).

The three carrier-removable silicone latex compositions from Dow Corning differ only slightly in material composition. Dow Corning 3-5024 is the base system containing hydroxyl endblocked dimethylpolysiloxane elastomer with 5 percent by weight $SiO_2$, and an anionic emulsifier and may also include a suitable cross-linking agent such as a silane and catalyst, such as an alkyl tin salt. This material is the least viscous (1Pa•s = 1000 cps) of the three and cures to a thin clear film.

A second silicone water based elastomer, Dow Corning 3-5025, identical to Dow Corning 3-5024 with the addition of an organic, thixotropic additive, has a precured viscosity of 25Pa•s (25000 cps). This film is also clear on drying.

A third silicone water based elatomer, Dow Corning 3-5035, includes about 4.5 percent by weight $TiO_2$ filler. These films ore opaque and white in color.

A heat-curable silicone paste (Dow Corning 3-9595) having essentially no volatizable carrier was also tested for comparison purposes. Dow Corning 3-9595 is a dimethylpolysiloxane elastomer containing 40 percent by weight silica and is supplied as a two-part putty-like material requiring the material to be spread into a layer using a doctor blade.

Results of the evaluation of membranes made from the above-identified four materials are summarized in the following table:

FLUX DENSITY (J) AND PERMEABILITY (P) OF SILICONE RUBBERS

| MATERIAL | GLUCOSE | URIC ACID | ASCORBIC ACID | $H_2O_2$ |
|---|---|---|---|---|
| DC3-5024 Clear Latex | J=1.78 X $10^{-11}$ | 7.45 X $10^{-14}$ | 5.79 X $10^{-14}$ | 1.60 X $10^{-12}$ |
| | P=7.31 X $10^{-9}$ | 2.12 X $10^{-10}$ | 2.04 X $10^{-10}$ | 6.88 X $10^{-9}$ |
| DC3-5025 Clear/ Latex | J=2.97 X $10^{-11}$ | 1.2 X $10^{-12}$ | 4.6 X $10^{-15}$ | 9.12 X $10^{-13}$ |
| | P=1.22 X $10^{-8}$ | 3.5 X $10^{-9}$ | 1.6 X $10^{-11}$ | 3.9 X $10^{-9}$ |
| DC3-5035 White/ Latex | J=8.49 X $10^{-11}$ | 3.6 X $10^{-12}$ | 6.9 x $10^{-14}$ | 2.3 X $10^{-11}$ |
| | P=3.49 X $10^{-8}$ | 9.8 X $10^{-9}$ | 2.5 X $10^{-10}$ | 9.9 X $10^{-8}$ |
| DC3-9595 putty consistency | J=7.41 X $10^{-13}$ | 1.5 X $10x^{-14}$ | 3.8 X $10^{-14}$ | 1.9 X $10^{-13}$ |
| | P=3.05 X $10^{-11}$ | 4.2 X $10^{-11}$ | 1.4 X $10^{-10}$ | 8.5 X $10^{-10}$ |

J = flux density (moles $cm^{-2}$ $sec^{-1}$)

P = permeability coefficient ($cm^2$ $sec^{-1}$)

Quite surprisingly, the glucose permeability of the silicone material in paste form having essentially no volatizable is three orders of magnitude lower than Dow Corning 3-5025 and Dow Corning 3-5035 and two orders of magnitude lower than Dow Corning 3-5024. The evaluation table also emphasizes the three latex materials are much more selective for glucose relative to ascorbic acid than the paste form silicone.

In the precured liquid state the silicone water-based elastomer may be coated over the substrate 14 and pads 12A and 12B by dipping the test device 10 into the liquid elastomer. The excellent adhesion

characteristics of the silicone elastomer eliminates the need for an adhesive to bond overcoat 20 to the substrate 14 and upon drying and curing, the overcoat 20 is securely bonded in three dimensions over substrate 14 and pads 12A and 12B. Overcoat 20, upon curing, adheres to the substrate 14 between pads 12A and 12B which inhibits runover of reactants between pads 12A and 12B. Runover between pads 12A and 12B can interfer with an accurate reading of the colors.

The silicone water-based elastomer cures to a stable, durable film 20 that may be rinsed and wiped without being damaged. This is a significant advantage since prior art membranes tend to change physically when exposed to moisture. The physical change in prior art membranes results in dimensional changes that may change the permeability resulting in inaccurate color indications of the concentration of glucose in the measured fluid. The physical change of prior art membranes also reduces their strength increasing the likelihood of tearing when wiped.

Contrary to prior art membranes, the overcoat 20 of the present invention remains stable after curing and does not change physically when exposed to moisture. Consequently, there is no change in strength or permeability in the overcoat 20 during performance of a test and red blood cells may be wiped off without damaging the overcoat 20.

Preferably, the thickness of overcoat 20 is controlled since the thickness influences the time necessary for a color changing reaction to occur. If overcoat 20 is too thick, the time for glucose to pass through the overcoat 20 and react with the color forming indicators in pads 12A and 12B will be too long. It has been determined a preferred thickness of overcoat 20 is 0.013 mm (one half mil). If the overcoat 20 is thinner than 0.013 mm (one half mil.), it can lose integrity and tend to tear if wiped.

Varying the thickness of overcoat 20, however, has advantages. As illustrated in FIG. 2, the thickness of overcoat 20 may be varied from end 16 to end 18 of the test device 10. The thickness of overcoat 20 over pad 12A may be thin or approximately 0.013 mm (one half mil) whereas the thickness of overcoat 20 over pad 12B is greater. With an overcoat 20 of varying thickness as illustrated, the time for glucose to penetrate overcoat 20 over pad 12A will be shorter than the time necessary to penetrate the thicker portion of overcoat 26 over pad 12B. The test device 10 with the varying thickness overcoat 20 provides a comparative type system. The higher the glucose concentration in the fluid being tested the faster a response will be registered since the diffusion rate of glucose through the overcoat 20 is proportional to the concentration of glucose in the sample being tested. For example, if there is a low concentration of glucose in the sample, after the sample fluid has been placed on device 10, the proper amount of time expired and the sample rinsed and wiped off the overcoat 20, only pad 12A will indicate a color. If there is a higher concentration, glucose will penetrate the thicker portion of overcoat 20 as well as the thinner portion and both pads 12A and 12B will indicate a color. The user may simply determine how many of the pads 12A and 12B indicate a color and this will represent the concentration of glucose in the sample being tested. The varying thickness overcoat 20 eliminates the need to compare the color of the pads 12A or 12B with a color chart.

## Claims

1. A multilayer test device for detecting glucose in a fluid, comprising:
   a first layer consisting of a liquid-retaining material impregnated with reagent capable of reacting with said glucose to produce a detectable change, and
   a second layer coating the first layer with a selectively glucose permeable, polymerized, silicone water-based elastomer.

2. The multilayer test device of claim 1 wherein said second layer is formed from a composition comprising a dispersed, polymerizable silicone water-based elastomer in a removable acquous carrier liquid, said silicone water-based elastomer being substantially insoluble in said carrier liquid, and a portion of said carrier liquid being removed during polymerization of said silicone water-based elastomer in dispersed, layered form.

3. The multilayer test device of any of claims 1 and 2 wherein the silicone water-based elastomer comprises an organosiloxane.

4. The multilayer test device of any of claims 1 to 3 wherein the silicone water-based elastomer comprises a diorganosiloxane.

5. A multilayer test article for testing for glucose in a fluid such as blood comprising:

a first layer of a bibulous material impregnated with a mixture including an enzyme system with glucose oxidase activity, a substance with peroxidative activity, and an indicator material which is oxidized in the presence of peroxide, said substance including peroxidative activity causing a change in colour, and
a second layer composed of a film of polymerized, silicone water-based elastomer bonded to the top of said bibulous material, said second layer being glucose permeable.

6. The multilayer test article according to claim 5 further comprising a substrate, said bibulous material comprising pads of said material mounted on said substrate and wherein said second layer is bonded over said pads and to said substrate between said pads.

7. The multilayer test article of any of claims 5 and 6 wherein said second layer comprises a glucose-permeable membrane formed from a layer of a dispersed, polymerizable silicone water-based elastomer in a removable carrier liquid, said silicone water-based elastomer being substantially immiscible in said carrier liquid, and a portion of said carrier liquid being removed during polymerization of said silicone water-based elastomer.

8. A multilayer test device fo determining glucose, comprising:
a first layer composed of a rigid support matrix, said matrix including a reactant system including at least one ingredient capable of producing a detectable response upon contact with said glucose,
the improvement comprising a second layer composed of a film of polymerized, silicone water-based elastomer of variable thickness over said first layer.

9. The multilayer test device set forth in claim 8 wherein said reactant system includes a plurality of pads of bibulous material and wherein said second layer is over and between said pads.

10. The multilayer test device according to any of claims 8 and 9 wherein said second layer is formed from a composition comprising a dispersed, polymerizable silicone water-based elastomer in a removable carrier liquid, said silicone water-based elastomer being substantially immiscible in said carrier liquid, and a portion of said carrier liquid being removed during polymerization of said silicone water-based elastomer in dispersed, layered form, said carrier liquid comprising at least 5% by weight of the layered composition prior to completion of polymerization.

## Revendications

1. Dispositif analytique multicouche pour la détection de glucose dans un liquide, comprenant :
une première couche constituée d'une matière retenant le liquide imprégnée d'un réactif capable de réagir avec le glucose pour produire une variation détectable, et
une seconde couche revêtant la première couche avec un élastomère de silicone à base d'eau polymérisé, sélectivement perméable au glucose.

2. Dispositif analytique multicouche suivant la revendication 1, dans lequel la seconde couche est formée d'une composition comprenant un élastomère de silicone à base d'eau polymérisable dispersé dans un véhicule liquide aqueux éliminable, ledit élastomère de silicone à base d'eau étant principalement insoluble dans ledit véhicule liquide et une portion du véhicule liquide étant éliminée pendant la polymérisation de l'élastomère de silicone à base d'eau sous forme de couche en dispersion.

3. Dispositif analytique multicouche suivant l'une quelconque des revendications 1 et 2, dans lequel l'élastomère de silicone à base d'eau comprend un organosiloxane.

4. Dispositif analytique multicouche suivant l'une quelconque des revendications 1 à 3, dans lequel l'élastomère de silicone à base d'eau comprend un diorganosiloxane.

5. Article analytique multicouche pour la recherche du glucose dans un liquide tel que le sang, comprenant :
une première couche d'une matière absorbante imprégnée d'un mélange renfermant un système enzymatique doué d'activité de glucose-oxydase, une substance douée d'activité de peroxydase et un

indicateur qui est oxydé en présence de peroxyde, ladite substance à activité de peroxydase provoquant un changement de couleur, et

une seconde couche composée d'un film d'élastomère de silicone à base d'eau polymérisé lié à la partie supérieure de la matière absorbante, la seconde couche étant perméable au glucose.

6. Article analytique multicouche suivant la revendication 5, comprenant en outre un substrat, ladite matière absorbante comprenant des tampons de cette matière montés sur ledit substrat et ladite seconde couche étant liée par-dessus lesdits tampons et audit substrat entre lesdits tampons.

7. Article analytique multicouche suivant l'une quelconque des revendications 5 et 6, dans lequel la seconde couche comprend une membrane perméable au glucose formée d'une couche d'un élastomère de silicone à base d'eau polymérisable dispersé dans un véhicule liquide éliminable, ledit élastomère de silicone à base d'eau étant principalement non miscible audit véhicule liquide et une portion du véhicule liquide étant éliminée pendant la polymérisation dudit élastomère de silicone à base d'eau.

8. Dispositif analytique multicouche pour la détection du glucose, comprenant une première couche composée d'une matrice de support rigide, ladite matrice comprenant un système de corps réactionnels renfermant au moins un ingrédient capable de produire une réponse détectable au contact dudit glucose,

le perfectionnement comprenant une seconde couche composée d'un film d'élastomère polymérisé de silicone à base d'eau d'épaisseur variable sur ladite première couche.

9. Dispositif analytique multicouche suivant la revendication 8, dans lequel le système de corps réactionnels comprend plusieurs tampons de matière absorbante et ladite seconde couche se trouve sur et entre lesdits tampons.

10. Dispositif analytique multicouche suivant l'une quelconque des revendications 8 et 9, dans lequel la seconde couche est formée d'une composition comprenant un élastomère de silicone à base d'eau polymérisable dispersé dans un véhicule liquide éliminable, ledit élastomère de silicone à base d'eau étant principalement non miscible dans ledit véhicule liquide et une portion du véhicule liquide étant éliminée pendant la polymérisation dudit élastomère de silicone à base d'eau sous forme de couche en dispersion, ledit véhicule liquide constituant au moins 5 % en poids de la composition en couche avant l'accomplissement de la polymérisation.

## Patentansprüche

1. Multischicht-Testvorrichtung zum Nachweisen von Glukose in einer Flüssigkeit, umfassend: eine erste Schicht, bestehend aus einem Flüssigkeit zurückbehaltenden Material, das mit einem Reagens imprägniert ist, das sich dazu eignet, mit der Glukose zu reagieren, wobei eine nachweisbare Änderung hervorgerufen wird, und eine zweite Schicht, die die erste Schicht mit einem gegenüber Glukose selektiv durchlässigen, polymerisierten Silikonelastomer auf Wasserbasis überzieht.

2. Multischicht-Testvorrichtung nach Anspruch 1, bei der die zweite Schicht aus einer Zusammensetzung gebildet ist, die ein dispergiertes, polymerisierbares Silikonelastomer auf Wasserbasis in einer entfernbaren, wässrigen Trägerflüssigkeit enthält, wobei das Silikonelastomer auf Wasserbasis im wesentlichen unlöslich in der Trägerflüssigkeit ist, und wobei ein Teil dieser Trägerflüssigkeit während der Polymerisation des Silikonelastomers auf Wasserbasis unter Ausbildung der dispergierten, geschichteten Form entfernt ist.

3. Multischicht-Testvorrichtung nach einem der Ansprüche 1 oder 2, bei der das Silikonelastomer auf Wasserbasis ein Organosiloxan enthält.

4. Multischicht-Testvorrichtung nach einem der Ansprüche 1 bis 3, bei der das Silikonelastomer auf Wasserbasis ein Diorganosiloxan enthält.

5. Multischicht-Testartikel zum Nachweisen von Glukose in einer Flüssigkeit, wie Blut, umfassend:

eine erste Schicht aus einem schwammigen Material, imprägniert mit einer Mischung, die ein Enzymsystem mit Glukoseoxidaseaktivität, eine Substanz mit Peroxidaseaktivität und ein Indikatormaterial, das in Anwesenheit von Peroxid oxidiert wird, umfasst, wobei die Substanz, die die Peroxidaseaktivität aufweist, eine Farbänderung bewirkt; und

eine zweite Schicht, die aus einem Film aus polymerisiertem Silikonelastomer auf Wasserbasis, das an die Spitze dieses schwammigen Materials gebunden ist, zusammengesetzt ist, wobei die zweite Schicht gegenüber Glukose durchlässig ist.

6. Multischicht-Testartikel nach Anspruch 5, der ferner ein Substrat umfasst, wobei das schwammige Material Kissen des Materials enthält, das an dem Substrat angebracht ist, und bei dem die zweite Schicht über diese Kissen und an das Substrat zwischen diese Kissen gebunden ist.

7. Multischicht-Testartikel nach einem der Ansprüche 5 und 6, bei dem die zweite Schicht eine für Glukose durchlässige Membran, gebildet aus einer Schicht aus einem dispergierten, polymerisierbaren Silikonelastomer auf Wasserbasis in einer entfernbaren Trägerflüssigkeit, umfasst, wobei das Silikonelastomer auf Wasserbasis im wesentlichen in der Trägerflüssigkeit unmischbar ist, und bei dem ein Teil der Trägerflüssigkeit während der Polymerisation des Silikonelastomeren auf Wasserbasis entfernt ist.

8. Multischicht-Testvorrichtung zum Nachweisen von Glukose, umfassend:

eine erste Schicht, zusammengesetzt aus einer steifen Trägermatrix, wobei diese Matrix ein Reaktantensystem umfasst, das mindestens einen Bestandteil, der sich dazu eignet, eine nachweisbare Reaktion nach Kontakt mit dieser Glukose zu erzeugen, enthält, wobei die Verbesserung eine zweite Schicht, zusammengesetzt aus einem Film aus polymerisiertem Silikonelastomer auf Wasserbasis von variierbarer Dicke über der ersten Schicht umfasst.

9. Multischicht-Testvorrichtung nach Anspruch 8, bei der das Reaktantensystem eine Vielzahl von Kissen aus schwammigem Material enthält, und bei dem die zweite Schicht sich über und zwischen diesen Kissen befindet.

10. Multischicht-Testvorrichtung nach einem der Ansprüche 8 und 9, bei der die zweite Schicht aus einer Zusammensetzung gebildet ist, die ein dispergiertes, polymerisierbares Silikonelastomer auf Wasserbasis in einer entfernbaren Trägerflüssigkeit enthält, wobei das Silikonelastomer auf Wasserbasis im wesentlichen in der Trägerflüssigkeit unmischbar ist, und ein Teil der Trägerflüssigkeit während der Polymerisation des Silikonelastomers auf Wasserbasis unter Ausbildung der dispergierten, geschichteten Form entfernt ist, wobei die Trägerflüssigkeit mindestens 5 Gew.% der geschichteten Zusammensetzung vor Vollendung der Polymerisation enthält.

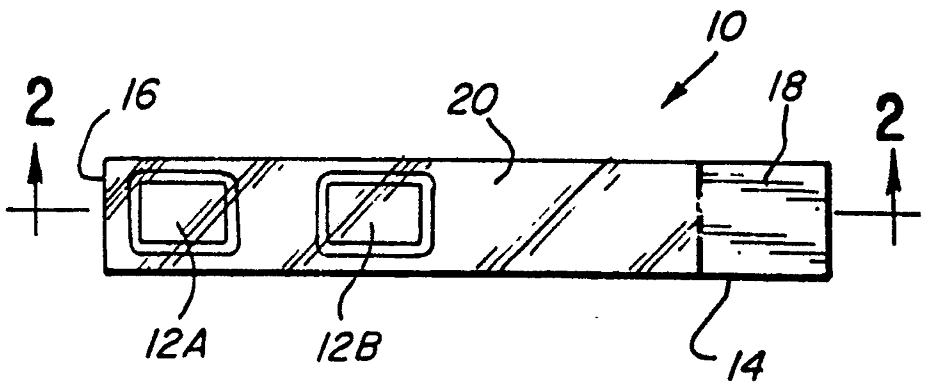


FIG. 1

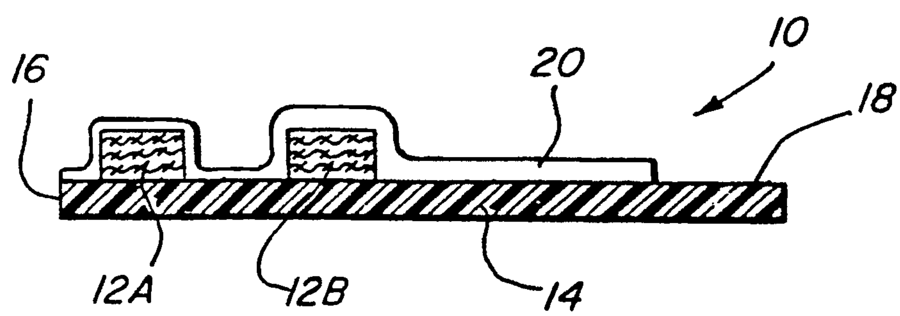


FIG. 2